# EUROPEAN PATENT APPLICATION

(11) **EP 2 330 418 A1**
(43) Date of publication of application: **08.06.2011**
(21) Application number: 09015066.5
(22) Date of filing: 04.12.2009
(51) Int. Cl.: G01N 33/50

(54) **Methods of quantitative determination of neurogenesis in vivo**

(71) Applicant: SYGNIS Bioscience GmbH & Co KG, 69120 Heidelberg (DE)
(72) Inventor: Schneider, Armin, 69118 Heidelberg (DE); Spoelgen, Robert, 69120 Heidelberg (DE); Meyer, Annette, 68723 Schwetzingen (DE)
(74) Representative: Lahrtz, Fritz

(57) **Abstract**

The present invention relates to methods for quantifying neurogenesis in vivo. These method combine the isolation of purified nuclei from the brain tissue, the specific labelling of the nuclei of newly generated neurons thereof, and the counting of these nuclei. Density sedimentation is suitable for the separation of the nuclei of the cells from the complex debris of the brain tissue. As a matter of course the markers which are used for that identification have to be presented on the surface of the nuclei or within the nuclei. FACS analysis is the preferred method for counting the differentially labelled nuclei of proliferating neurons and the ones of other neural cells.

## Description

Loss of neurons in the brain and the low ability to replace dying neurons is a hallmark of various disorders of the nervous system. Whereas it seemed impossible to compensate this loss of brain cells for a long time, we know today that the nervous system has the ability to regenerate nerve cells throughout the lifetime of humans and other mammals. The events related to the generation of new nerve cells have been termed neurogenesis.

To develop new therapeutic strategies which stimulate the generation of neurons in the adult brain, it is essential to measure neurogenesis in animals by fast and reliable techniques. Particularly, it is important to measure the neurogenic effect of drug candidates or other stimuli in vivo in animal models. As of today, identification of newly generated neurons is mainly based on histological techniques, which are labour intensive and extremely time consuming.

The identification of newly generated neurons is mainly based on cell labelling with certain marker proteins, such as NeuN, doublecortin DCX, tubulin ßIII (clone TuJ-1), MAP2b, and others (Maric and Barker, Mol Neurobiol 2004, 30:49-76; Maurer and Kuschinsky, Curr Stem Cell Res Ther 2006, 1:65-77). Conventional methods employ standard immunocytochemistry or immunohistochemistry, with a low spatial resolution and the limitation to process large number of sections as required for industrial screenings.

Recently, transgenic animals have been generated, which carry reporter genes (like EGFP) under the control of neurogenic sensitive promoters like the doublecortin promoter (WO 2004/067751). Yet, it is unclear whether the insertion of these construct itself influence the neurogenesis or whether these genetic modifications influence the effect of therapeutic compounds on neurogenesis.

For in vivo tracing of transplanted stem cells, methods based on magnetic resonance imaging (MRI) have been developed (West et al., Exp Neurol 2007, 203:22-33; Hoehn et al., Proc Natl Acad Sci USA 2002, 99:16267-16272). This technology only allows the tracing of transplanted stem cells and not the analysis of endogenous neurogenesis.

An alternative approach to quantify neurogenesis is microarray analysis, based on the fact that gene expression varies between cell types and cell status. The disadvantage of gene expression profiling is low temporal resolution, but even more limiting, animals need to be analyzed in a specific time frame after treatment before changes in gene expression are back to normal levels. After all, gene expression in one cell type may be masked by opposing expression in another cell type. Especially in the mammalian brain, where different neuronal and non-neuronal cells are highly mixed, microarray analysis can give false-positive or false-negative results.

Fluorescent-activated cell sorting (FACS) is a convenient alternative to count cells in various applications. FACS based analyses of fluorescence labelled proliferating neural cells have be described before (Bilsland et al., J Neurosci Methods 2006, 157:54-63; Rahman et al., Society for Neuroscience, 2008, Poster 558.8, DD19). However, the complexity of the brain tissue makes this approach extremely challenging. The neural cells are highly interweaved with each other which makes it virtually impossible to separate the cells without disruption and yielding in cells contaminated with debris of other cell types.

There is a need for improved means and methods for measuring neurogenesis which meets the needs in the pharmaceutical research and, thereby, for identifying drug candidates with neurogenic effect for the treatment of neurodegeneration or impaired or insufficient neurogenesis. The embodiments characterized in the claims and herein below provide such means.

Accordingly, the present invention relates to a combined counting of newly generated cells (e.g. by flow cytometry measurement) with a preceding purification protocol yielding the isolated cell nuclei. Such purification protocol can be based on density sedimentation which enables the separation of the nuclei of the cells from the complex debris of the brain tissue. In a second step, the isolated nuclei are characterized by immunostaining to identify neuronal proliferation. As a matter of course the markers which are used for that identification have to be presented on the surface of the nuclei or within the nuclei. FACS analysis is the preferred method for counting the differentially labelled nuclei of proliferating neurons and the ones of other neural cells. However, other nuclei counting methods are also suitable, e.g. counting the differentially labelled nuclei within a suspension under a fluorescence microscope, preferably by automatic devises.

In contrast to previous approaches to prepare the brain tissue for FACS measurement, the isolation of purified cell nuclei of the brain tissue according to the invention ensures a clean separation of FACS countable events from the complex debris of the brain tissue. FACS measurements using whole cells or unpurified homogenates of nuclei and cell debris can not cope with the complexity of the brain tissue and present no reliable tool to screen high numbers of animals.

The technique described herein is not restricted to measure neurogenesis in brain tissue, but allows to be adopted for cell culture experiments, accordingly.

The person skilled in the art knows various purification protocols for the isolation of pure and intact cell nuclei. Within a first step, the brain tissue is disrupted in order to release the cell nuclei (Herculano-Houzel and Lent, J Neurosci 2005, 25:2518-2521). This can be done for example with a Dounce homogenizer as described in Example 1. Eventually, the homogenate is exposed to a fixative such as 4 % para-formaldehyd. Thereafter, the cell nuclei are separated from the debris. Suitable methods for separating the cell nuclei are for example density sedimentation or gel permeation chromatography. Preferably, density sedimentation is used for the separation of the cell nuclei as described in Example 1. The density sedimentation can be performed by ultracentrifugation and provides a clean fraction of nuclei that allows proper staining and counting. In order to obtain a clean fraction of nuclei which allows reproducible counting with a low variance of replicates, the percentage of nuclei in the samples preferably exceed 25 % of all counted particles of similar size (e.g. nuclei, cell debris and impurities). This ratio (pure nuclei per all counted particles (particularly cell debris)) can be measured by FACS methods known in the art using DNA-labelling compounds like 7-amino-actinomycin D (7-AAD) or propidium iodide for staining the nuclei as described exemplarily in Example 4.

According to the invention, the purified cell nuclei of proliferating neurons have to be fluorescently labelled to enable the subsequent - preferably FACS based - quantification of neurogenesis. This can be done by labelling suitable markers which are expressed in or on the nuclei of that newly generated neurons. Usually, marker specific antibodies which are coupled to fluorescent dyes or combinations of specific primary antibody and fluorescently labelled secondary antibodies are used for the labelling. Alternatively, one can use transgenic animals which express fluorescent proteins in the nucleus (e.g. Green fluorescent Protein (GFP)) under the control of promoters activated in premature or newly generated neurons. Suitable neurogenesis markers according to the invention have to be presented at least partially on the surface or within the nuclei.

During neurogenesis neural stem cells differentiate initially to premature neurons which subsequently develop further to mature neurons. The labelling of marker proteins (e.g. doublecortin (Couillard-Despres et al., Eur J Neurosci 2005, 21:1-14) , sox1 (Barraud et al., Eur J Neurosci 2005, 22:1555-1569), stathmin (Jin et al., FASEB Journal 2004, 18:287-299)) which are specifically expressed by such premature neurons is suitable to analyze the extent of neurogenesis.

Alternatively, newly developed cells can be generally labelled by administering Bromodeoxyuridine (BrdU) or derivatives thereof, which are incorporated into the DNA of that cells during cell division. The labelling of such incorporated BrdU in combination with the labelling of neuronal marker proteins (e.g. NeuN, Musashi-1 (Sakakibara et al., Dev Biol 1996, 176:230-242) or Sox2 (Suh et al., Cell Stem Cell 2007, 1:515-528)) allows for the identification of neurons which have been developed by neurogensis during the period of BrdU administration. Advantageously, that method allows for a precise determination of the chronological progress of the neurogenesis in vivo in response to certain stimuli (e.g. exercise or administration of test compounds) within an experimental setting.

For the proper identification of neurogenesis, it is crucial to discriminate between newly generated neuronal and non neuronal cells. To that end, only cells labelled for both BrdU and NeuN are considered to quantify neurogenesis.

Neurogenesis is known to occur in discrete regions of the adult brain, including the rostral subventricular zone (SVZ) of the lateral ventricles and the subgranular zone (SGZ) of the hippocampal dentate gyrus (DG). Therefore, the hippocampal tissue is a preferred source for the assessment of neurogenesis according to the present invention. Of cause, the methods of the present invention are also suitable to measure neurogenesis in other regions of the brain as well. The methods of the present invention can be also used for comparing the neurogenic potential of the various brain regions.

Suitable buffers for the nuclei purification by density sedimentation using ultracentrifugation are known in the art. Preferably, such buffers is a salt buffer of physiological concentration containing an ingredient to adjust the density such as sucrose or Polyethylene glycol (PEG). Suitable buffers according to the invention are provided in Example 1.

The ratio of the count of the specifically labelled nuclei of newly generated neurons versus the count of the nuclei of all neural cells is a suitable measure for neurogenesis according to this invention. By "neural cells" are meant all brain tissue cells including neurons and glial cells.

Neurogenesis is one mechanism that can lead to increased plasticity of neural networks, and can replace gradual loss of neurons. Therefore, neurogenesis can provide enhancement, improvement, or an increase in cognitive ability to an individual suffering from, displaying, and/or believed to some level of cognitive loss by administering compositions with neurogenesis stimulating and therefore neuroregenerative effect. Advantageously, a neuroregenerative acting compound or composition allows for the treating of a neurological condition even at a late stage after progressive neuronal loss. Such a compound enables to reverse neuronal loss which have had already occurred. By contrast a compound which solely acts neuroprotective can be used only to stop or attenuate the ongoing process of neuronal dying.

Neurogenesis stimulating compounds or compositions can be used to treat neurological conditions with a need for neuroregeneration. These neurological conditions comprise cerebral ischemia (such as stroke, traumatic brain injury, or cerebral ischemia due to cardiocirculatory arrest), amyotrophic lateral sclerosis (ALS), glaucoma, Alzheimer's disease, neurodegenerative trinucleotide repeat disorders (such as Huntington's disease), neurodegenerative lysosomal storage diseases, multiple sclerosis, spinal cord injury, spinal cord trauma, dementia, schizophrenia, and peripheral neuropathy. Additionally, said compounds can be used to enhance learning and memory, e.g. in the case of non-pathological conditions such as age-related memory loss, mild cognitive impairment, or age-related cognitive decline.

Neurogenesis stimulating compounds or compositions can be for example polypeptides, nucleic acids, or small molecules. Preferably for treating neurodegenerative diseases such compounds are small molecules, more preferably with a molecular weight of less than 2,000 g. Compound libraries either with well-known drug candidates or with proprietary substances are available and can be used as source for the identification of neurogenesis stimulating compounds based on the analysis method according to this invention. Compound libraries are usually generated by combinatorial chemistry or by collecting known substances. To assess the neurogenic potential the compounds have to be administered to the test animal and subsequently the brain tissue has to be analyzed according to the invention for enhanced neurogenesis compared to sham treated control animals. A test compound is regarded as neurogenic according to the invention if neurogenesis is enhanced in treated animals by at least 20 %, compared to control animals and/or if neurogenesis is enhanced statistically significantly in treated animals compared to control animals. Whether a portion is statistically significant can be determined without further ado by the person skilled in the art using various well known statistic evaluation tools, e.g., determination of confidence intervals, p-value determination, Student's t-test, Mann-Whitney test etc. Preferred confidence intervals are at least 95 %. The p-values are, preferably, 0.05.

The route of administration of such test compounds can be the typical routes including, for example, orally, subcutaneously, transdermally, intradermally, intramuscularly, intravenously, by direct injection to the brain, and parenterally.

Neurogenesis can be enhanced not only by administration of neurogenic compounds but also by certain stimuli such as exercise. Actually, exercise is known to be a comparably strong stimulus of neurogenesis (Chen et al., Neurosci Bull 2006, 22:1-6; Van der Borght et al., Behav Neurosci 2007, 121:324-323; van Praag et al., Nat Neurosci 1999, 2:266-270). Therefore, the present invention is also suitable to test and compare the neurogenic effect of stimuli such as variants of exercises.

The methods of the present invention can be used for in vivo assessing of neurogenesis in animals, preferably in mammals such as mice or rats. Healthy animal can be used within the methods in order to study the neurogenic impact of a test stimulus under normal conditions. However, the present methods are also suitable to analyse potentially altered neurogenesis or neurogenic impact of test stimuli within animal models for neurological conditions and diseases.

Animal models for certain neurological conditions and diseases which are suitable for the methods according to this invention, particularly, for the quantitative evaluation of the neurogenic effect of test compounds in vivo are well known in the art. Such a model is for example the rat MCAO model (middle cerebral occlusion; filament model) which is an animal model for stroke, where a broad variety of different neuron types is damaged by ischemia/hypoxia. Further animal models are for example but not limited to SOD1G93A-transgenic mouse for amyotrophic lateral sclerosis (Almer et al., J Neurochem 1999, 72:2415-2425), APP-transgenic mice for Alzheimer's disease (Janus & Westaway, Physiol Behav 2001, 73:873-886), MPTP-treated animals for Parkinson's disease (Smeyne & Jackson-Lewis, Brain Res Mol Brain Res. 2005, 134:57-66), exon-1-huntingtin-transgenic mice for Huntington's disease (Sathasivam et al., Philos Trans R Soc Lond B Biol Sci. 1999, 354:963-969), and FRDA-transgenic mice for Friedreich ataxia (Al-Mahdawi Genomics 2006, 88:580-590). The measurement of neurogenesis of the animals treated with the test compound have to be compared with sham treated animals in order to identify compounds which exhibit neuroregenerative effect and therefore can be beneficial for the outcome of the diseases.

Accordingly, the present invention provides methods for quantitative determination of neurogenesis in vivo, which comprise
(a) the disruption of neural tissue or cell aggregations and, thereby, the release of the corresponding cell nuclei,
(b) the purification of the nuclei from the cell debris,
(c) the differential labelling of nuclei of newly generated neuronal cells in contrast to the nuclei of the other neural cells using neurogenesis specific and nuclei located markers, and
(d) the counting of said nuclei of newly generated neuronal cells.

In one embodiment of above methods, the purification of the nuclei is done by density sedimentation using an ultracentrifuge.

Preferably, the percentage of the nuclei in the purified fraction exceed 25 % of all particles of similar size.

Also in one embodiment of above methods, the ratio of the nuclei of newly generated neuronal cells versus the nuclei of all neural cells serves as measure for neurogenesis.

In a further embodiment of above methods, the specific labelling of nuclei of newly generated neuronal cells is attained by administering BrdU at the beginning of the experiment and by using the combination of newly incorporated BrdU and the neuronal protein NeuN as markers for said nuclei of newly generated neuronal cells.

Additionally, in one embodiment of above methods, the counting of the differentially labelled nuclei is done by FACS methods.

Preferably, the methods of the present invention are used for the assessment of the neurogenic effect of test stimuli in vivo. Accordingly, in one embodiment of the present invention said methods are used for identifying or evaluating neurogenic substances and other stimuli. Accordingly, these methods can be used to evaluate e.g. the neurogenic efficiency of rehabilitation therapies after or during neurological conditions. Preferably, two or more test animals per group (with and without exposing to the test stimulus) were used for such an assessment to allow for statistical evaluation.

Substances and other stimuli which revealed to be neurogenic by means of these methods can be used for treating neurodegenerative diseases. Such substances can be used for preparation of pharmaceutical compositions for treating neurodegenerative diseases.

Also one embodiment of the invention is a kit which comprises at least suitable buffers for homogenizing brain tissue and purifying nuclei thereof. Preferably, such a kit also comprises suitable labelling reagents to allow for a differential staining of nuclei of newly generated neurons and nuclei of other neural cells, such as anti-BrdU antibody, anti-NeuN antibody and 7-AAD.

### Brief description of the figures

Figure 1:
   Homogenized hippocampal brain tissue without and with purification protocol analysed by phase contrast microscopy (100-fold magnification). Tissue homogenate before the density sedimentation shows a high content of cell debris (A). Tissue homogenate after purification presents a nuclei fraction (B, arrows mark nuclei) which is applicable for fluorescent staining of nuclei according to the invention.
Figure 2:
   Nuclei purified according to Example 1 and immunostaind according to Example 2 were analysed by fluorescence microscopy for neuronal identity by NeuN (A) and for the proliferation marker BrdU (B), also nuclei were counterstained with 7-AAD (C). The arrow marks a nuclei of a newly generated neuron which is double labelled for NeuN and BrdU.
Figure 3:
   FACS-based analysis of neurogenesis in mice that underwent physical training (A) in comparison to animals without exercise (B) according to Example 3. Nuclei stained for BrdU and NeuN are depicted in a dot plot whereas each counted event is located according to the intensity of its BrdU label (FITC-BrdU fluorescence on the x-axis (log scale), newly generated cells with higher FITC-BrdU fluorescence on the right hand side) and of its NeuN label (PE-NeuN fluorescence on the y-axis (log scale), neurons with higher PE-NeuN fluorescence on the upper side). The lower right area comprises the FACS events corresponding to newly developed but non-neuronal cells (BrdU-positive but NeuN-negative), whereas the upper right area comprises the ones corresponding to newly generated neurons (NeuN-positive and BrdU-positive).
Figure 4:
   Hippocampal neurogenesis of mice given access to exercise in order to stimulate neurogenesis ("exercise" group, n=7) are compared with control mice without this stimulus ("untreated" group, n=7). Neurogenese is measured by the FACS based analysis of cell nuclei according to the invention as described in Example 3. The number of nuclei of newly generated - BrdU-positive - neuronal cells (per 100,000 nuclei) and the number of nuclei of the newly developed - BrdU-positive and NeuN-positive - neurons (per 100,000 nuclei) are represented by the hatched and white columns, respectively. Presented are the mean values and the standard deviation. Animals which had access to exercise on a running wheel showed significantly more double labelled (BrdU- and NeuN-positive) nuclei (p-value < 0,05 based on Student's t-test). The method of the invention clearly reveals the enhanced neurogenesis (ratio of BrdU- and NeuN-positive cells) due to the stimulation by access to exercise.
Figure 5:
   Comparison of FACS based analysis after conventional tissue preparation without nuclei purification with FACS based analysis after tissue preparation of the invention including nuclei purification. The percentage of measurable events (nuclei per all counted particles (e.g. nuclei, cell debris and impurities)) from the brain tissue by FACS analysis is about 27 times higher when using the tissue preparation of the invention including nuclei purification in contrast to an enzymatic dissociation of cells without nuclear extraction (A, column 1: tissue preparation of the invention including nuclei purification; column 2: conventional tissue preparation without nuclei purification). The FACS counts of NeuN labelled (PE fluorescent) nuclei after conventional tissue preparation (B) and after tissue preparation of the invention (C) is plotted against PE fluorescence intensity (x-axis, log scale) within histograms. The nuclei purification of the invention allows a proper dissection between neuronal (NeuN positive) and non neuronal (NeuN negative) character, whereas the conventional technique barely allows the quantification of the neuronal character due to low amount of measurable events and inhomogeneous preparation. The neurogenesis analyses based on conventional techniques depend very much on an arbitrarily set discrimination between labelled and unlabelled FACS events and, thereby, are error-prone.
Figure 6:
   Evaluation of the FACS-based analysis by comparison to the well established histological analysis. Neurogenesis was analyzed in three different groups of animals (n=10, each): mice that underwent physical exercise (E); mice treated with the neurogenic drug Thiabendazol (T) and control animals (C). Within the histological analysis the counts of BrdU positive and of BrdU and NeuN double positive cells in the dentate gyrus per mm³ were determined. Within the FACS based method according to the invention BrdU positive and BrdU and NeuN double positive nuclei per 100,000 nuclei were counted. Represented are mean values and standard error of means as error bar (hatched columns: BrdU positive; white columns: BrdU and NeuN double positive). Table 1 summarizes the means and standard errors of means. Both techniques - the technique according to the invention and the conventional histological technique - showed comparable results to measure neurogenesis, as BrdU and NeuN double positive events were increased by 36 % or 23 %, respectively, by the pharmacological intervention or 8 times or 4 times, respectively, by physical training compared to controls. The FACS based technique according to the invention performed with lower variance compared to the histological technique. Therefore, only the FACS based technique according to the invention revealed a significantly enhanced neurogenesis due to the Thiabendazol administration (p < 0.05, Student's t-test), whereas under the same conditions the conventional histological technique failed to fulfil these statistic significance criteria.

**Table 1:**

| | Control | Thiabendazol administration | physical exercise |
|---|---|---|---|
| Histological technique | | | |
| (positive cells per mm³ in dentate gyrus) | | | |
| BrdU positive (mean) | 58955 | 69782 | 208804 |
| BrdU positive (SEM) | 4621 | 7497 | 13822 |
| BrdU/NeuN double positive (mean) | 35526 | 43665 | 141315 |
| BrdU/NeuN double positive (SEM) | 3117 | 5026 | 9402 |
| | | | |

| FACS based technique of the inventions | | | |
|---|---|---|---|
| (positive nuclei counts per 100,000 total nuclei counts | | | |
| BrdU positive (mean) | 878 | 883 | 1509 |
| BrdU positive (SEM) | 60 | 35 | 72 |
| BrdU/NeuN double positive (mean) | 69 | 94 | 551 |
| BrdU/NeuN double positive (SEM) | 7 | 8 | 35 |

### Examples:

### Example 1

### Preparation of a purified cell nuclei fraction from brain tissue.

Animals were anaesthetised and brains isolated. Hippocampi (or other relevant tissue) were removed from brain using forceps and scalpel. Hippocampi were then minced and transferred into nuclei dounce homogenizer. Dounce homogenizer was used to homogenize hippocampi in salt buffer of physiological concentration containing 0.32 M sucrose, 15 mM NaCl, 60 mM KCl, 15 mM HEPES, 3 mM MgCl₂, 2 mM EDTA, and 50 mM Spermidine at pH 6.8 (buffer A). The homogenates were washed once and fixed in 4 % para-formaldehyde (PFA) for 30 min. After fixation, the homogenates were washed and resuspended in above-mentioned salt buffer but having 0.9 M sucrose. The homogenates were centrifuged twice in this buffer and the supernatants were removed to separate the crude nuclear pellet from cell debris. The crude nuclear pellet was resuspended in 400 µl of buffer A and carefully pipetted onto a layer of 1 ml of above-mentioned salt buffer but having 1.3 M sucrose. The nuclei were then centrifuged in an ultracentrifuge (Beckmann, Optima TLX)) for 45 min at 50,000 g. The purified nuclei were recovered as a small white pellet on the bottom of the tube.

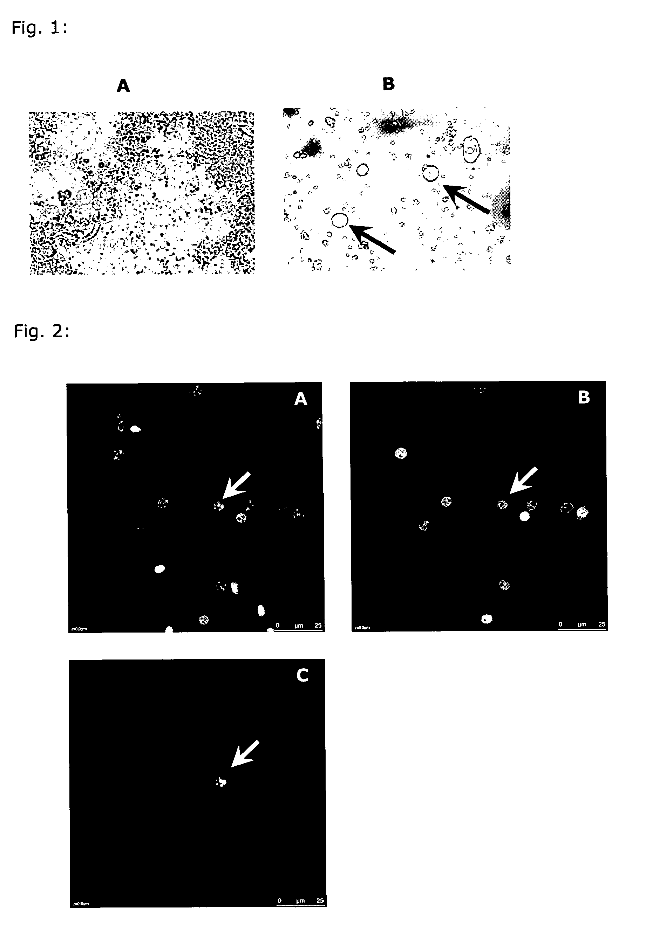

The purity of the cell nuclei fraction was proven by visual inspection with a microscope (Fig. 1).

### Example 2

### Fluorescent labelling of neural cell nuclei and flow cytometry analysis thereof.

To access the extent of neurogenesis in test animals, animals were analysed three weeks after the last BrdU injection. Neural cell nuclei were isolated as described in Example 1. In order to detect BrdU labelled nuclei the FITC-BrdU flow cytometry kit (BD Bioscience, Heidelberg) was used.

The nuclei were fixed and permeablised employing cytofix and cytoperm buffer (BD Bioscience, Heidelberg). To denaturate DNA, nuclei were incubated with DNase (300 mg/ml in PBS buffer) for 60 min at 37 °C. Nuclei were then washed and incubated with FITC conjugated BrdU antibody solution. After that labelling step nuclei were washed once with PBS and incubated with mouse anti-Neuronal Nuclei (NeuN) biotin conjugated antibody (MAB377B, Chemicon, Hampshire, UK). For fluorescence labelling of NeuN, nuclei were washed with PBS and the PE conjugated streptavidin (BD Pharmingen, San Diego CA, USA) was employed. Finally, nuclei were washed twice with PBS and treated with 7-AAD (Biomol, Plymouth Meeting, PA, USA) before flow cytometry measurement.

Fig. 2 shows fluorescence microscopy of such labelled nuclei.

Flow cytometry was undertaken using FACS Calibur™ system (BD Bioscience, Heidelberg), containing a single argon ion-laser and 5 detector channels. For analysis 100,000 events were visualised on a forward side scatter plot and gated for 7-AAD positive events. Analyses were based on the FITC- and PE-fluorescence and gates were controlled by the analysis of animals which were not treated with BrdU.

### Example 3

### Analysis of the extent of adult neurogenesis in animals

Adult neurogenesis was induced in adult mice by exercise, which is known to promote neurogenesis in the hippocampus. One group of animals (n=7) was given access to a running wheel over 3 weeks. A second group (n=7) which had no access to a running wheel served as the control group. To label newly generated cells, animals were treated with BrdU in the first five days of the experiment. After three weeks hippocampi were dissected from mice and cell nuclei were purified as described in Example 1. Subsequently, the purified nuclei were processed for FACS measurement according to Example 2.

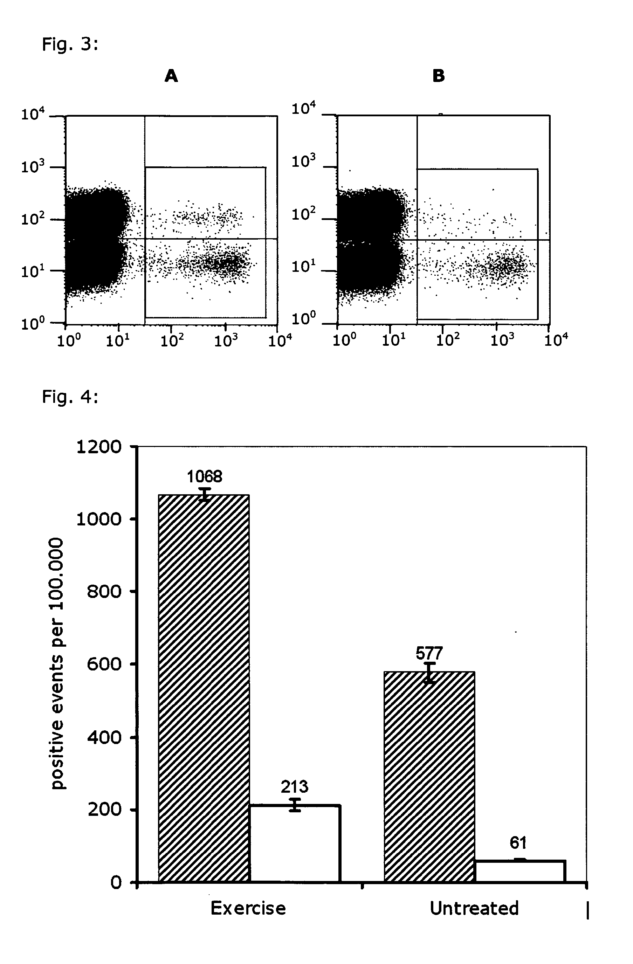

The FACS measurement of the nuclei after NeuN- and BrdU-labelling allows for the quantification of newly generated neurons which comprise NeuN as well as BrdU labels. This fraction is clearly separated from the nuclei of neurons that already existed before the stimulation (NeuN label but no BrdU label) and from the nuclei of non-neuronal newly developed brain cells (BrdU but no NeuN label) (Fig. 3).

The validity of the method according to the invention for measuring neurogenesis is demonstrated by the comparison of exercised and untreated animals. Significant differences in neurogenesis were detected between both groups (p-value < 0.05 based on Student's t-test). The exercise group showed 0.21 % newly generated neurons (of all cells) in the hippocampus compared to 0.06 % newly generated neurons in the control group (Fig. 4).

That neurogenesis assay according to the invention allowed for measuring neurogenesis about 8 times faster than conventional histological techniques (counting cells in tissue slices using fluorescence microscopy), with the additional advantage to process multiple samples in parallel.

### Example 4

Comparison of FACS based analysis after conventional tissue preparation without nuclei purification with FACS based analysis after tissue preparation of the invention

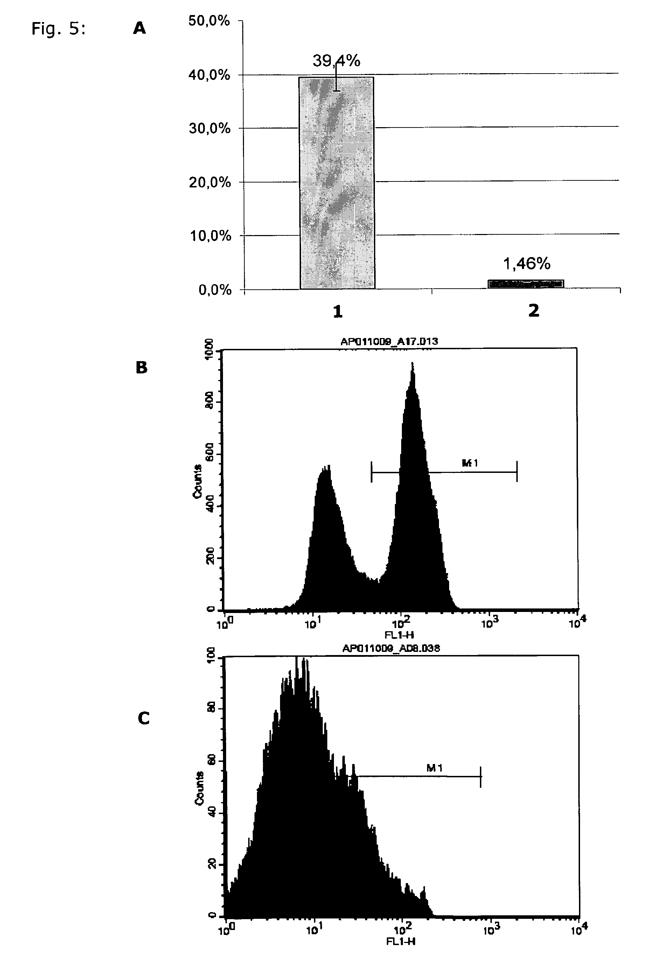

FACS analysis was performed according to Example 2 after tissue preparation of the invention as described within Example 1. A corresponding FACS analysis after the same tissue preparation but without the nuclei purification served as control. Such conventional tissue preparations without the nuclei purification have been previously reported (Bilsland et al., J Neurosci Methods 2006, 157:54-63 or Herculano-Houzel and Lent, J Neurosci 2005, 25:2518-2521). The percentage of measurable events (nuclei per all counted particles (e.g. nuclei, cell debris and impurities)) was determined by 7-AAD staining of the nuclei and standard FACS method. This ratio was approximately 40 % for the technique of the invention which was about 27 times of the corresponding ratio of the control (Fig. 5 A). The FACS counts of NeuN labelled (PE fluorescent) nuclei after the conventional tissue preparation (Fig. 5 C) and after the tissue preparation of the invention (Fig. 5 B) has been determined as a function of PE fluorescence intensity. The nuclei purification of the invention allows a proper dissection between neuronal (NeuN positive) and non neuronal (NeuN negative) character, whereas the conventional technique barely allows the quantification of the neuronal character due to low amount of measurable events and inhomogeneous preparation. The results auf neurogenesis analyses based on such a conventional techniques depend very much on an arbitrarily set discrimination between labelled and unlabelled FACS events and, thereby, are error-prone.

### Example 5

Evaluation of the FACS-based analysis by comparison to the well established histological analysis.

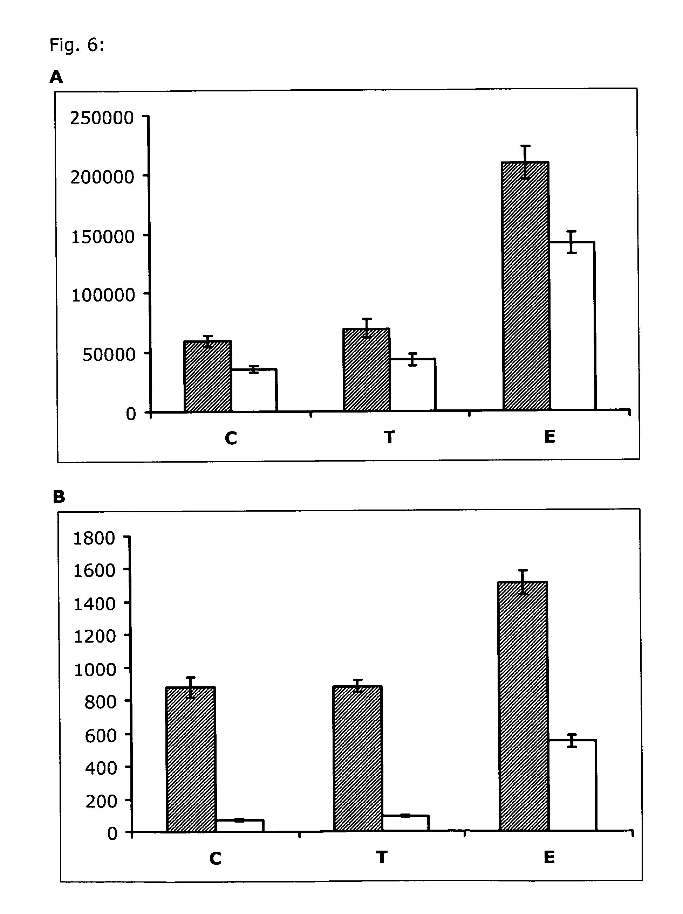

Neurogenesis was analyzed in three different groups of animals (n= 10, each group), namely mice that underwent exercise as described in Example 3, mice treated with the neurogenic drug Thiabendazol (three weeks of oral administration via nutrition pellets (555 mg Thiabendazol per kg pellets to allow for a daily dose of 100 mg per kg body weight), and control animals. Neurogenesis was analyzed by the technique according to the invention (as described in Example 3) and in parallel by conventional histological technique. For the histological technique 7 paraffin embedded hippocampal sections (10 µm, sagittal, 340 µm section-to-section distance) per animal were prepared in order to cover the complete dentate gyrus. The sections were fluorescently stained for the markers BrdU and NeuN using the primary antibodies anti-BrdU antibody (Abcam, Cambridge, MA, USA) and anti-NeuN antibody (Millipore, Billerica, MA, USA) and secondary antibodies coupled with fluorescent dyes. Additionally, the sections were counter-stained with hemalaun. Subsequently, the newly generated neural cells (BrdU positive) and the newly generated neurons (BrdU and NeuN double positive) in the area of dentate gyrus and the volume of the dentate gyrus were stereologically determined based on the 7 sections per animal using the computer program Mercator (ExploraNova, La Rochelle, France) and the fluorescence microscope IX81 (Olympus, Germany) in order to attain the number of newly generated cells in the dentate gyrus per volume. Both techniques - the technique of the invention and the conventional histological technique - showed comparable results to measure neurogenesis, as BrdU and NeuN double positive events were increased by 36 % or 23 %, respectively, by the pharmacological intervention or 8 times or 4 times, respectively, by physical training compared to controls (Fig. 6). However, the conventional histological technique is much more labour-intensive. Also the histological technique failed to show a significant neurogenic effect for the pharmacological intervention due to higher variance of the measurements. In contrast, the technique of the invention resulted in a smaller variance and therefore the neurogenic effect of the pharmacological intervention measured by this method revealed to be significant (p < 0.05, Student's t-test).

## Claims

1. A method for quantitative determination of neurogenesis in vivo, which comprises
a. the disruption of neural tissue or cell aggregations and, thereby, the release of the corresponding cell nuclei,
b. the purification of the nuclei from the cell debris,
c. the differential labelling of nuclei of newly generated neuronal cells in contrast to the nuclei of the other neural cells using neurogenesis specific and nuclei located markers, and
d. the counting of said nuclei of newly generated neuronal cells.

2. The method of claim 1, wherein the purification of the nuclei is done by density sedimentation.

3. The method of claim 1 or 2, wherein the counting of the differentially labelled nuclei is done by FACS methods.

4. The method of any of the claims 1 to 3, wherein the percentage of the nuclei in the purified fraction exceed 25 % of all particles of similar size.

5. The method of any of the claims 1 to 4, wherein the ratio of the nuclei of newly generated neuronal cells versus the nuclei of all neural cells is used as measure for neurogenesis.

6. The method of any of the claims 1 to 5, wherein the specific labelling of nuclei of newly generated neuronal cells is attained by administering BrdU at the beginning of the experiment and by using the combination of newly incorporated BrdU and the neuronal protein NeuN as markers for said nuclei of newly generated neuronal cells.

7. A method for assessing of the neurogenic effect of a test stimulus within test animal in comparison to control animal without said test stimulus, which comprises
a. exposing animal to the test stimulus,
b. quantitative determination of neurogenesis within test animal and control animal by a method of any of the claims 1 to 6, and
c. comparison of the quantitative determination of neurogenesis of test animal versus control animal.

8. The method of claim 7, wherein the test stimulus is the administration of a test substance.

9. The method of claim 7, wherein the test stimulus is a physical therapy.
